# EUROPEAN PATENT SPECIFICATION

(11) **EP 3 606 402 B1**
(45) Date of publication and mention of the grant of the patent: **05.07.2023**
(21) Application number: 18710963.2
(22) Date of filing: 27.02.2018
(51) Int. Cl.: A61B 1/018, A61M 39/06

(54) **A MEDICAL VALVE WITH A VARIABLE DIAMETER SEAL**
MEDIZINISCHES VENTIL MIT EINER DICHTUNG VARIABLEN DURCHMESSERS
VALVE MÉDICAL AVEC UN JOINT D'ÉTANCHÉITÉ AU DIAMÈTRE VARIABLE

(30) Priority: 16.06.2017 US 201715624755
(43) Date of publication of application: 12.02.2020
(73) Proprietor: Freudenberg Medical, LLC, Carpinteria, CA 93013 (US)
(72) Inventor: FURNISH, Greg, Louisville KY 40206 (US); APPLING, Anthony, Crestwood KY 40014 (US); MORRIS, Ben, Jeffersonville IN 47130 (US); FURNISH, Simon, Louisville KY 40206 (US)
(74) Representative: Manitz Finsterwald Patent- und Rechtsanwaltspartnerschaft mbB
(86) International application number: PCT/US2018/019854
(87) International publication number: WO 2018/190961

(56) References cited:
- EP-A1- 2 965 685
- US-A1- 2012 238 958
- US-B1- 6 458 103

## Description

### CROSS-REFERENCE TO RELATED APPLICATIONS

This application is a continuation-in-part of U.S. Patent Application No. 15/624,755, filed on June 16, 2017, which is a continuation-in-part of U.S. Patent Application No. 15/483,089 filed on April 10, 2017, which is a continuation of U.S. Patent Application No. 14/326,593, now U.S. Patent No. 9,616,213, filed on July 9, 2014.

### BACKGROUND OF THE INVENTION

### 1. Field of the Invention

The present disclosure relates generally to medical devices and procedures. In particular, the present disclosure relates to hemostatic valves and systems, and methods of using the same. EP2965685 discloses a medical valve assembly according to the state of the art.

### 2. Description of the Prior Art

This section provides background information related to the present disclosure which is not necessarily prior art.

Numerous procedures have been developed that involve the percutaneous insertion of a medical device into a body vessel of a patient, with the medical device being introduced into the vessel by a variety of known techniques. Each of these procedures must control the flow of bodily fluids when the medical device is inserted into the body vessel. Accordingly, medical valves, such as hemostatic valves, iris valves, laproscopic ports, or the like, are often used to limit or prevent blood loss during the procedure.

Hemostatic valves often incorporate a disk valve to control fluid flow through the medical device. However, disk valves are subject to deformation with both time and use, and often can tear or become dislodged during insertion and/or withdrawal of the medical device. Furthermore, disk valves are not designed to provide an effective seal across a wide range of differently sized medical devices. Although the disk valve can be modified to accommodate these situations, such as with increased tensile and/or elongation properties, this modification leads to increased resistance, and thus require the use of excessive force, when the medical device is inserted and withdrawn through the disk valve.

Iris valves can include an elastomeric sleeve that is disposed within a valve body and which is interconnected to a rotatable cap. When the cap is rotated in a first direction, an opening extending through the elastomeric sleeve is opened. Conversely, when the cap is rotated in a second opposite direction, the elastomeric sleeve is twisted and constricted to effectuate a closure of the elastomeric sleeve. However, if the operator stops the rotation, the elastomeric sleeve can revert, or recoil, back to the open position. Additionally, even when the elastomeric sleeve is held in the closed position, gaps or channels extend therethrough as a result of the twisting or infolding required to effectuate a closure. Accordingly, fluid can leak through the iris valve in the closed position. Further, the continuous twisting and constricting of the elastomeric sleeve leads to wear of the sleeve, such as through tearing.

The drawbacks associated with the existing medical valves are further exemplified when one considers that a single medical valve often is used to insert multiple medical devices during a single procedure. For example, a hemostatic valve may be used first for introducing a delivery catheter, followed by an interventional catheter. In this example, the hemostatic valve must be able to provide a hemostatic seal under a variety of conditions, i.e., accommodate a variety of different sized medical devices. Additionally, the hemostatic valve device must be able to quickly adjust to use of each of these different medical devices, otherwise significant fluid loss can occur through the medical valve.

### SUMMARY OF THE INVENTION

This section provides a general summary of the disclosure and is not intended to be a comprehensive disclosure of its full scope, aspects, objectives, and/or all of its features. The invention is defined according to claim 1, the dependent claims defining favourable embodiments.

A medical valve assembly for use in inserting a medical device into a body vessel of a patient includes a tube extending between a first tube end and a second tube end to define a passageway extending longitudinally along an axis between the ends. A plunger plate extends radially from the second tube end and a valve housing surrounds the tube about the second tube end. The valve housing extends from a first valve housing end to a second valve housing end and includes a housing flange extending radially inwardly from the second valve housing end, with the housing flange disposed in spaced relationship with respect to the plunger plate so as to define a distance dimension therebetween. An elastomeric seal is compressed between the plunger plate and the housing flange and has an inner diameter for use in establishing a variable seal of the medical valve assembly. A compression member is disposed within the valve housing and is biased against the plunger plate for decreasing the inner diameter to establish a closed inner diameter of the elastomeric seal and a respective closed condition of the medical valve assembly. A pair of lever arms are pivotably connected to the valve housing and radially compressible to overcome the bias of the compression member and effectuate axial movement of the valve housing relative to the tub to increase the distance between the plunger plate and the housing flange and increase the inner diameter of the elastomeric seal from the closed condition. A locking ring is rotatably connected to the second valve housing end and is rotatable relative to the pair of lever arms to establish and maintain a radially compressed condition of the pair of lever arms and a respective increased inner diameter of the elasteromic seal. As a result, the locking ring allows a user of the medical valve assembly to establish and maintain the increased inner diameter of the elastomeric seal during use without the need for the user to constantly maintain a manual radial compression on the pair of lever arms. Thus, the locking ring improves the user experience during use of the medical valve assembly.

Further areas of applicability will become apparent from the description provided herein. The description and specific examples in this summary are intended for purposes of illustration only and are not intended to limit the scope of the present disclosure.

### BRIEF DESCRIPTION OF THE DRAWINGS

The drawings described herein are for illustrative purposes only of selected embodiments, and are not all possible implementations and thus are not intended to limit the scope of the present disclosure.
Figure 1 is an environmental view of a first embodiment of a medical valve constructed in accordance with the principles of the present disclosure and illustrating a user interacting therewith;
Figure 2 is an environmental view of a second embodiment of the medical valve constructed in accordance with the principles of the present disclosure and illustrating the user interacting therewith;
Figure 3 is a perspective view of the first embodiment of the medical valve illustrating a scissor-type manual actuator;
Figure 4A is a cross-sectional view of the first embodiment of the medical valve illustrating a closed condition;
Figure 4B is a cross-sectional view of the first embodiment of the medical valve illustrating an open condition;
Figure 5 is a partial view taken from Figure 3 illustrating an elastomeric seal of the medical valve;
Figure 6 is a cross-sectional view of the first embodiment of the medical valve illustrating an alternative arrangement for the manual actuator;
Figure 7 is a perspective view of the first embodiment illustrating a detachable cap disposed over a pair of lever arms associated with the scissor-type manual actuator;
Figure 8 is a perspective view of the second embodiment of a medical valve constructed in accordance with the present disclosure;
Figure 9 is a cross-sectional view of the second embodiment shown in Figure 8;
Figure 10 is a perspective view of the first embodiment illustrating an alternative arrangement of the scissor-type manual actuator;
Figure 11 is a perspective view of a medical valve illustrating a first arrangement of a locking ring in accordance with the principles of the present disclosure and disposed in a first, closed position;
Figure 12 is a cross-sectional view of the medical valve of Figure 11;
Figure 13 is a perspective view of the medical valve illustrating the first arrangement of the locking ring disposed in the second, open position;
Figure 14 is a cross-sectional view of the medical valve of Figure 13;
Figure 15 is a perspective view of the medical valve illustrating a second arrangement of a locking ring in accordance with the principles of the present disclosure and disposed in a first, closed position;
Figure 16 is a perspective view of the second arrangement of the locking ring illustrating a pair of locking flanges each defining a locking surface that presents a plurality of steps;
Figure 17 is a perspective view of the medical valve illustrating the second arrangement of the locking ring disposed in a first intermediate position;
Figure 18A is a cross-sectional perspective view of the medical valve of Figure 17 illustrating a first intermediate inner diameter established and maintained by the first intermediate position of the locking ring;
Figure 18B is a cross-sectional end view of the medical valve illustrating a first step of the plurality of steps disclosed in engaging relationship with a respective one of the lever arms in the first intermediate position of the locking ring;
Figure 19 is a perspective view of the medical valve illustrating the second arrangement of the locking ring disposed in a second intermediate position;
Figure 20A is a cross-sectional perspective view of the medical valve of Figure 19 illustrating a second intermediate inner diameter established and maintained by the second intermediate position of the locking ring;
Figure 20B is a cross-sectional end view of the medical valve illustrating a second step of the plurality of steps disclosed in engaging relationship with a respective one of the lever arms in the first intermediate position of the locking ring;
Figure 21 is a perspective view of the medical valve illustrating the second arrangement of the locking ring disposed in the open position;
Figure 22A is a cross-sectional perspective view of the medical valve of Figure 21 illustrating an open inner diameter established and maintained by the open position;
Figure 22B is a cross-sectional end view of the medical valve illustrating a third step of the plurality of steps disclosed in engaging relationship with a respective one of the lever arms in the open position of the locking ring;
Figure 23 is an exploded perspective view of the medical valve illustrating a third arrangement of the locking ring in accordance with the principles of the present disclosure;
Figure 24 is a cross-sectional view of the medical valve illustrating the third arrangement of the locking ring disposed in an open position;
Figure 25 is a cross-sectional view of the medical valve illustrating the third arrangement of the locking ring disposed in a first intermediate position;
Figure 26 is a cross-sectional view of the medical valve illustrating the third arrangement of the locking ring disposed in a second intermediate position; and
Figure 27 is a cross-sectional view of the medical valve illustrating the third arrangement of the locking ring disposed in a closed position.

### DESCRIPTION OF THE ENABLING EMBODIMENTS

Example embodiments will now be described more fully with reference to the accompanying drawings. The example embodiments are provided so that this disclosure will be thorough and fully convey the scope to those skilled in the art. Numerous specific details are set forth such as examples of specific components, devices, mechanisms, assemblies, and methods to provide a thorough understanding of various embodiments of the present disclosure. It will be apparent to those skilled in the art that specific details need not be employed, that example embodiments may be embodied in many different forms, and that neither should be construed to limit the scope of the disclosure. With this in mind, the present disclosure is generally directed to medical valve assemblies of the type used to introduce and withdrawal a medical device (i.e., a guide wire, catheter, stent, filter, etc.) into a body vessel of a patient. In particular, each of the medical valve assemblies of the present disclosure incorporate a variable seal arrangement and a manually-operable actuator for controlling an entry dimension of the variable seal arrangement.

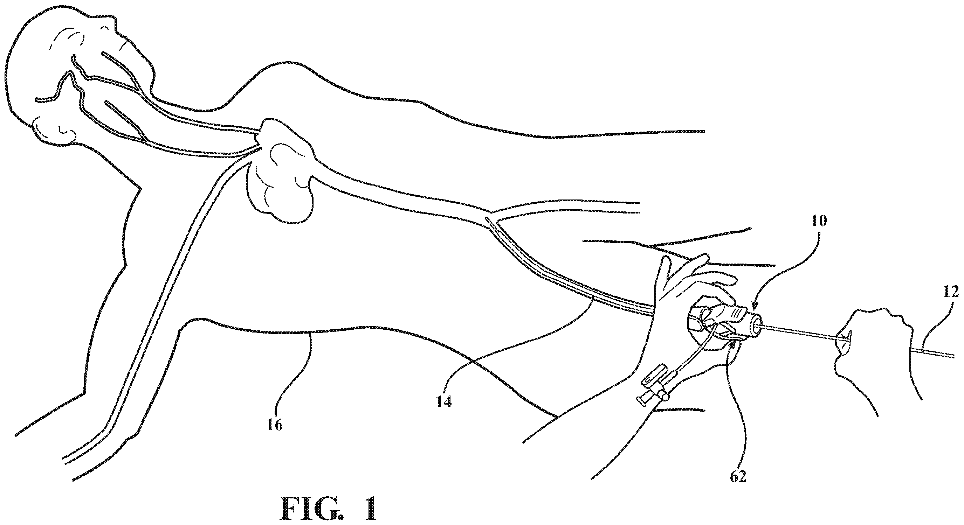

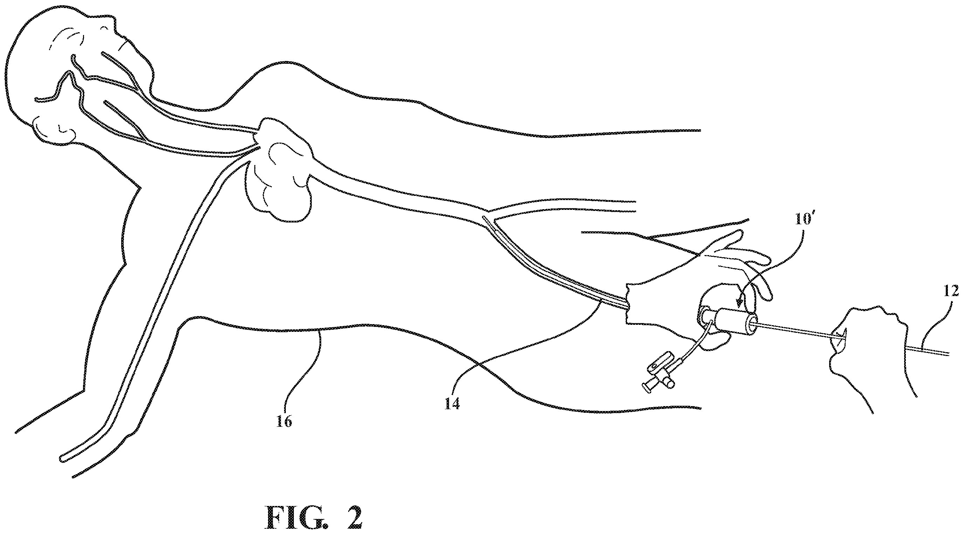

Referring to the Figures, wherein like numerals indicate corresponding parts throughout the several views, an environmental view of a first embodiment of a medical valve assembly **10** and a second embodiment of a medical valve assembly **10'** is generally shown in Figures 1 and 2, respectively. As illustrated therein, each medical valve assembly **10, 10'** is of the type for use with a medical device **12,** such as a guide wire, catheter, stent, filter, vessel occlusion device, or the like. As will be explained in more detail below, as the medical device **12** is inserted and guided through the medical valve assembly and into a body vessel **14** of a patient **16,** a user can manually actuate or interact with the medical valve assembly to effectuate a variable seal with variety of different sized medical devices **12.**

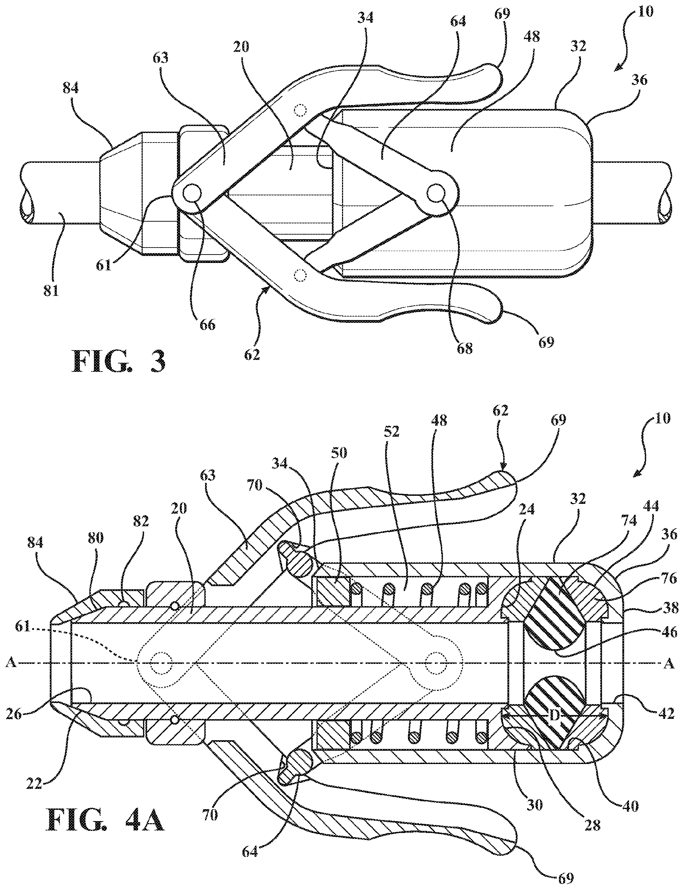

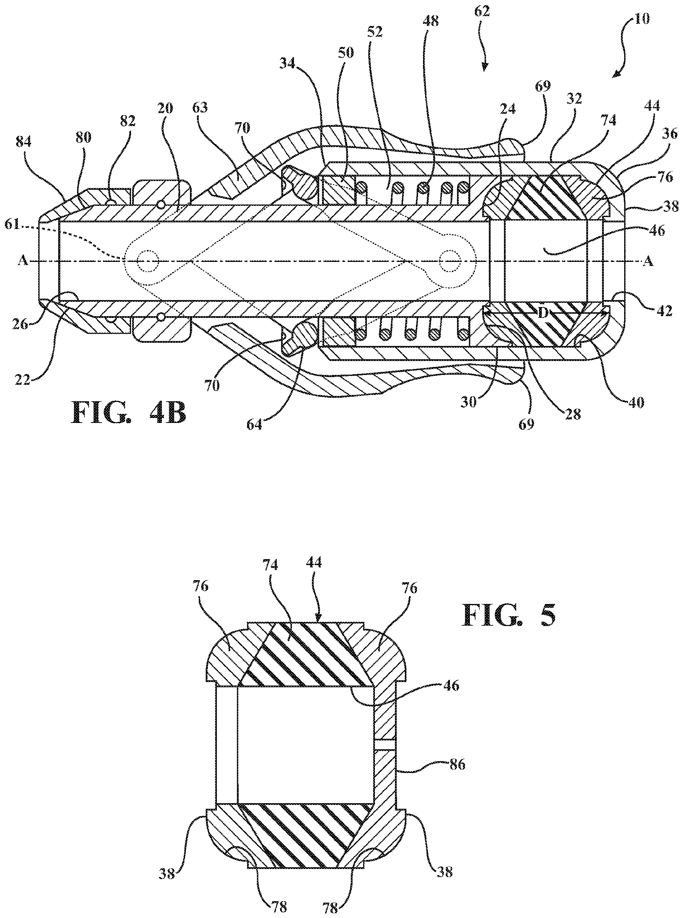

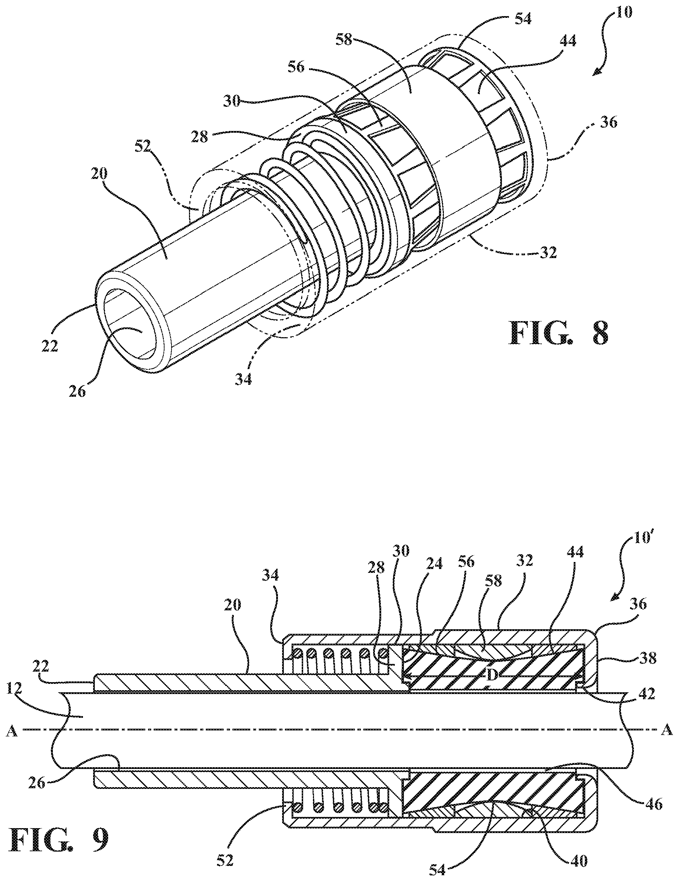

As best shown in Figures 4A, 4B and 9, the medical valve assemblies **10, 10'** each include a tube **20** extending between a first tube end **22** and a second tube end **24** to define a passageway **26** extending longitudinally along an axis **A** between the ends **22, 24,** with the passageway **26** being sized to receive a variety of differently sized medical devices **12.** In this instance, the first tube end **22** is a distal tube end and the second tube end is a proximal tube end **24.** A plunger plate **28** extends radially from the second tube end **24** to define an outer plunger plate surface **30** extending in spaced and parallel relationship to the axis **A.** A valve housing **32** is disposed in surrounding relationship with the tube **20** about the second tube end **24** and extends from a first valve housing end **34** to a second valve housing end **36** to overlay the outer plunger plate surface **30.** In this instance, the first valve housing end **34** is a distal valve housing end and the second valve housing end **36** is a proximal valve housing end **36.** As best shown in Figures 4A, 4B and 9, the valve housing **32** is disposed in spaced and parallel relationship with the tube **20** between the first valve housing end **34** and the plunger plate **28.**

The valve housing **32** includes a housing flange **38** extending radially inwardly from the second valve housing end **36.** The housing flange **38** is disposed in spaced relationship with the plunger plate **28** to define a distance dimension **D,** as well as a cavity **40,** extending therebetween. The housing flange **38** also defines an opening **42** aligned on the axis **A** and that is sized to receive a variety of differently sized medical devices **12.** An elastomeric seal **44** is installed in the cavity **40** and normally is pre-loaded or compressed between the plunger plate **28** and the housing flange **38.** The elastomeric seal **44** is used to establish a variable seal of the medical valve assembly **10, 10'.** In both of the first and second embodiments of the medical valve assembly **10, 10',** one of the valve housing **32** or the tube **20** is axially movable relative to the other to vary the distance dimension **D** between the plunger plate **28** and the housing flange **38** for effectuating an adjustment of an inner diameter **46** of the elastomeric seal **44.** In other words, the axial movement of one of the valve housing **32** or the tube **20** relative to the other results in a change in the compression load exerted on the elastomeric seal **44** which, in turn, allows the inner diameter **46** of the elastomeric seal **44** to be varied or adjusted in size. As best shown in Figures 4A, 4B and 9, when the valve housing **32** or the tube **20** is axially moved, the plunger plate **28** or the valve housing **32** axially slides relative to the other along the outer plunger plate surface **30.** In other words, the outer plunger plate surface **30** guides a sliding axial movement between the valve housing **32** and the tube **20.**

As best shown in Figures 4A, 4B, and 9, a compression member **48, 54** is disposed within the valve housing **32** and is compressed against the plunger plate **28** for normally closing or decreasing the inner diameter **46** to establish a closed position of the elastomeric seal **44.** As a result, the compression member **48, 54** is arranged to effectuate a closing or decreasing of the inner diameter **46** of the elastomeric seal **44** to establish a closed condition of the medical valve assembly **10, 10'.** In its closed condition, the elastomeric seal **44** completely isolates or seals the opening **42** of the valve housing **32** from the passageway **26** of the tube **20.** The valve housing **32** or the tube **20** is then axially movable relative to the other to alter a distance **D** between the housing flange **38** and the plunger plate **28** and shift the medical valve assembly **10, 10'** from the closed condition to an open/operative condition. The altered or varied distance **D** between the housing flange **38** and the plunger plate **28** allows the elastomeric seal **44** to expand, and as a result, the inner diameter **46** of the elastomeric seal **44** is expanded or increased to move the elastomeric seal **44** from its closed position to an open position. With the elastomeric seal **44** in its open position, the medical device **12** is positioned to be inserted serially through the opening **42,** the inner diameter **46** of the elastomeric seal **44** and the passageway **26** of the medical valve assembly **10.**

As best shown in Figures 4A and 4B, in the first embodiment of medical valve assembly **10,** the compression member **48, 54** comprises a coil spring **48** radially disposed between the valve housing **32** and the tube **20** and compressed between the first valve housing end **34** and the plunger plate **28.** However, any other suitable compression member could be utilized without departing from the scope of the subject disclosure. In a preferred embodiment, a disk **50** is slidably disposed around the tube **20** and interconnected to the first valve housing end **34** to establish a shoulder **52** extending radially inward from the valve housing **32** and which is disposed in engagement with the coil spring **48.** The coil spring **48** acts to bias the valve housing **32** towards the first tube end **22** for compressing the elastomeric seal **44** between the housing flange **38** and the plunger plate **28** and normally position the elastomeric seal **44** in its closed position. The valve housing **32** is then axially movable from the closed position and relative to the tube **20** to increase the distance **D** between the housing flange **38** and the plunger plate **28.** The increased distance **D** allows the elastomeric seal **44** to expand in an increased area of the cavity **40** disposed between the housing flange **38** and the plunger plate **28,** and as a result, the inner diameter **46** of the elastomeric seal **44** is expanded or increased, thereby opening the elastomeric seal **44.** The result is the establishment of the open condition of the medical valve assembly **10.**

As best shown in Figures 8 and 9, in the second embodiment of the medical valve assembly **10',** the compression member **48, 54** also comprises a coil spring **48** radially disposed between the valve housing **32** and the tube **20** and compressed between the first valve housing end **34** and the plunger plate **28.** However, any other suitable compression member could be utilized without departing from the scope of the subject disclosure. The valve housing **32** defines a shoulder **52** extending radially inward from the first valve housing end **34** and slidably disposed around the tube **20.** The shoulder **52** is disposed in engagement with the coil spring **48,** and the coil spring **48** acts to bias the valve housing **36** towards the first tube end **22.** In a preferred embodiment, the compression member **48, 54** additionally includes a leaf spring cage **54** disposed in surrounding relationship with the elastomeric seal **44.** However, any other suitable compression member could be utilized without departing from the scope of the subject disclosure. The leaf spring cage **54** extends between the plunger plate **28** and the housing flange **38** and is compressed therebetween by way of the compression spring **48.** The leaf spring cage **54** includes a plurality of struts **56** each extending axially along the leaf spring cage **54** and configured to fold radially inward towards the elastomeric seal **44** when the valve housing **36** is axially biased towards the first tube end **22** by the compression spring **48.** As a result, the distance **D** between the plunger plate **28** and the housing flange **38** is decreased, thus causing the elastomeric seal **44** to compress and reduce the inner diameter **46.** Put another way, the coil spring **48** and the leaf spring cage **54** interact to compress the elastomeric seal **44** between the housing flange **38** and the plunger plate **28** and normally position the elastomeric seal **44** in its closed position. As a medical device **12** is inserted through the passageway **26,** the medical device **12** engages the elastomeric seal **44** with an insertion force that is transferred or exerted radially outward on the struts **56** of the leaf spring cage **54,** causing the leaf spring cage **54** to expand and counteract the biasing force of the coil spring **48.** As a result, the distance between the plunger plate **28** and the housing flange **38** is increased, allowing the inner diameter **46** of the elastomeric seal **44** to expand or increase and establish the open condition of the medical valve assembly **10'.** A constrictor band **58** extends around the leaf spring cage **54** to prevent the plurality of struts **56** from engaging the valve housing **32** when the leaf spring cage **54** is expanded by the insertion force of the medical device **12.**

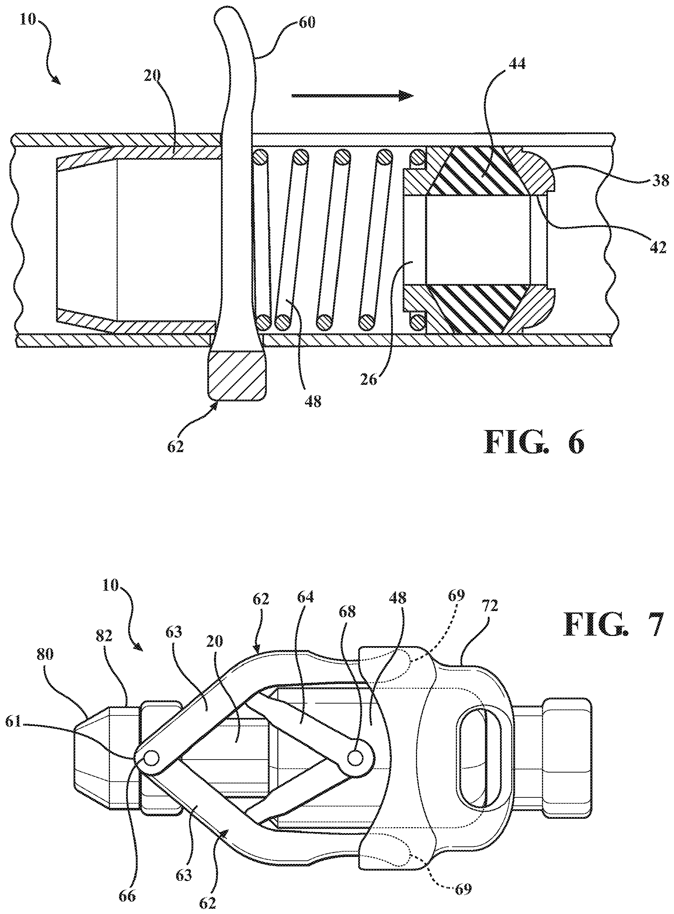

As best shown in Figures 3, 4A, and 4B, the first embodiment of the medical valve assembly **10** includes a manual actuator **62** which can be connected to the valve housing **32** for allowing a user to interact with the medical valve assembly **10** and vary a size of the inner diameter **46** of the elastomeric seal **44.** Put another way, the user can interact with the manual actuator **62** to overcome the bias of the compression member **48** and move the valve housing **32** relative to the tube **20** along the axis **A** towards the second tube end **24.** As a result, the manual actuator **62** allows the user to manually establish the open condition of the medical valve assembly **10.** As best shown in Figure 6, in the alternative embodiment, the manual actuator **62** can include a trigger arm **60** extending radially from the valve housing **32.** In this situation, the user can pull back on the trigger arm **60** to establish the open condition of the medical valve assembly **10.** In other words, a user can pull back the trigger arm **60** to vary the bias on the plunger plate **28.**

As best shown in Figures 3 and 4, the manual actuator **62** can include a pair of lever arms **63** interconnected between the tube **20** and the valve housing **32** by way of a pair of lever linkages **64.** As best shown in Figure 3, each lever arm **63** extends from a first lever arm end **61** pivotably connected to the tube **20** via a first pivot **66** extending radially from the tube **20** to a second lever arm end **69** having an arcuate shape relative to said axis **A.** Each lever linkage **64** includes a second pivot **68** extending radially from the valve housing **32.** The pair of lever linkages **64** are pivotably connected to the valve housing by the second pivots **68** with each of the lever linkages **64** extending from the respective second pivot **68** to engage one of the respective lever arms **63.** As best shown in Figure 7, in a preferred embodiment, each of the lever arms **63** can also define a track **70** for receipt of the respective lever linkage **64** when the lever linkages **64** are disposed in abutting relationship with the lever arms **63.** This arrangement of the lever arms **63** and the lever linkages **64** allows the user to squeeze or compress the pair of lever arms **63** with a specific force to axially advance the valve housing **32** by way of the lever linkages **64.** As a result, the transferred force effectuates the increase in the distance **D** between the plunger plate **28** and the housing flange **38,** and thus the increase in the inner diameter **46** of the elastomeric seal **44.** Put another way, a user can radially squeeze or compress the lever arms **63** to release compression on the elastomeric seal **44** and increase the inner diameter **46** of the elastomeric seal **44** from the closed condition to a desired size of the inner diameter **46** based on an amount of radial squeeze.

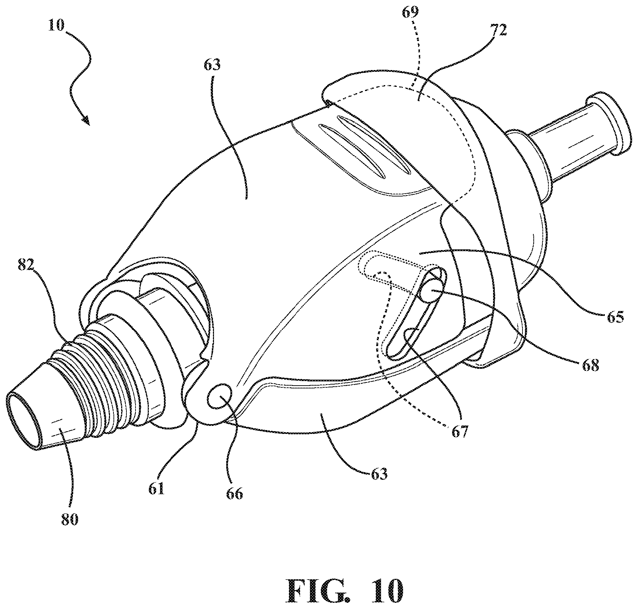

As best shown in Figure 10, in an alternative arrangement the pair of lever arms **63** can be interconnected between the tube **20** and the valve housing **32** by way of a pair of plates **65.** In a preferred embodiment, each lever arm **63** includes a plate **65** which extends radially therefrom and which defines a cam slot **67** for receiving the second pivot **68** extending radially from the valve housing **32.** This arrangement of the lever arms **63** and the plates **65** allows the user to squeeze or compress the pair of lever arms **63** with a specific force to slide the second pivot **68** along the cam slots **67** and axially advance the valve housing **32** by way of the plates **65.** As a result, the transferred force effectuates the increase in the distance **D** between the plunger plate **28** and the housing flange **38,** and thus the increase in the inner diameter **46** of the elastomeric seal **44.** Put another way, a user can radially squeeze or compress the lever arms **63** to release compression on the elastomeric seal **44** and increase the inner diameter **46** of the elastomeric seal **44** from the closed condition to a desired size of the inner diameter **46** based on an amount of radial squeeze.

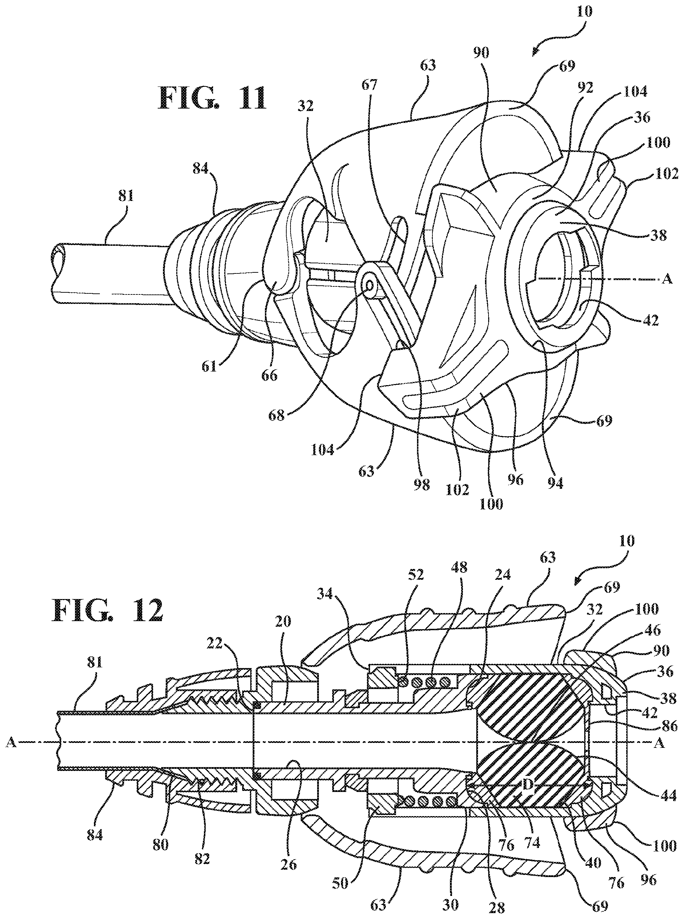

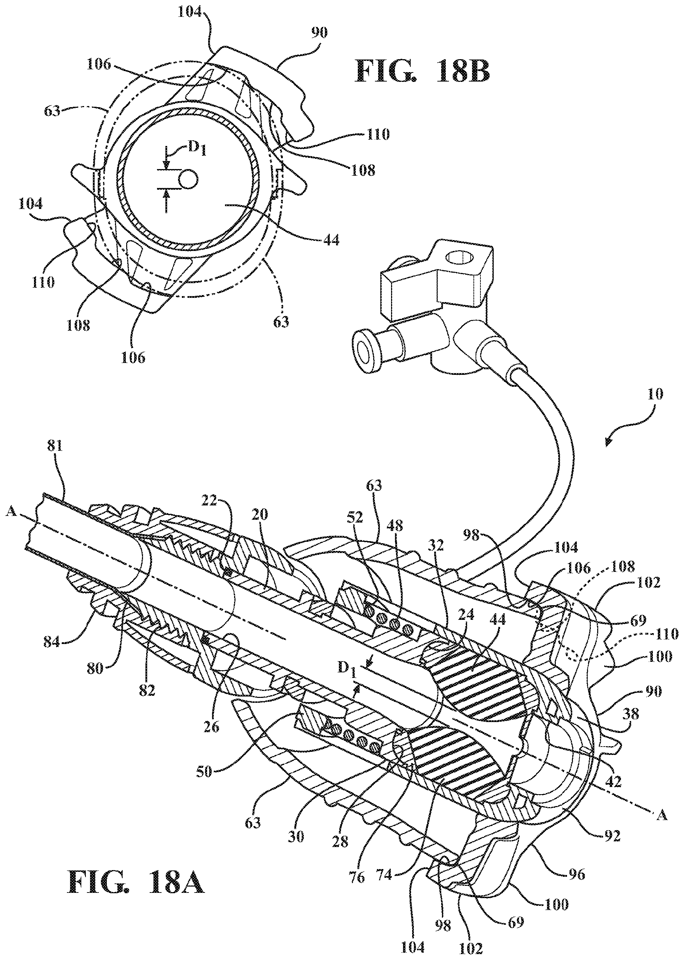

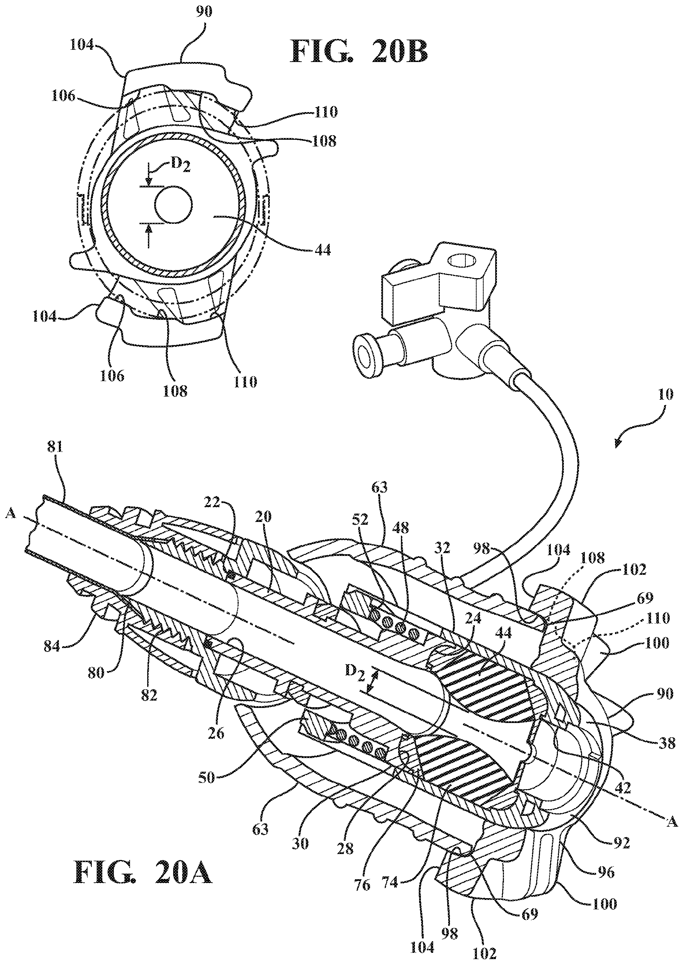

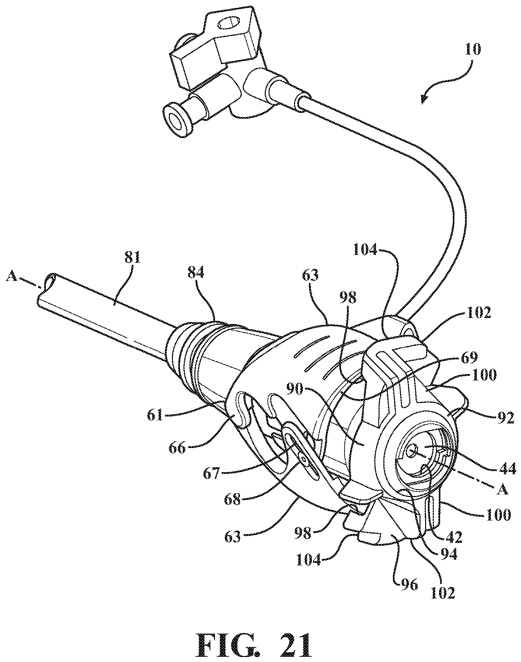

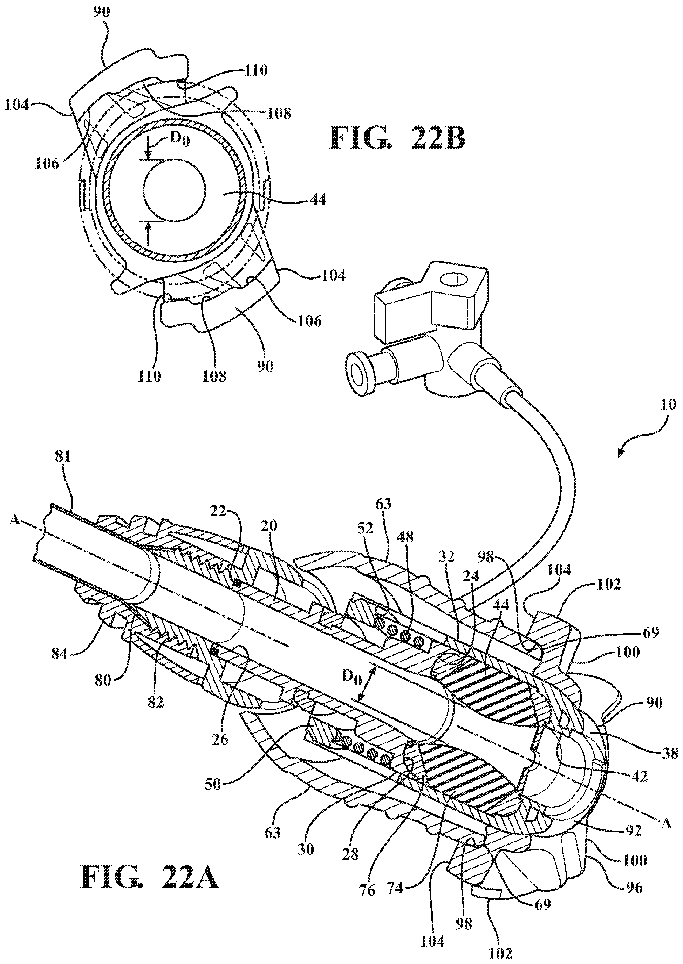

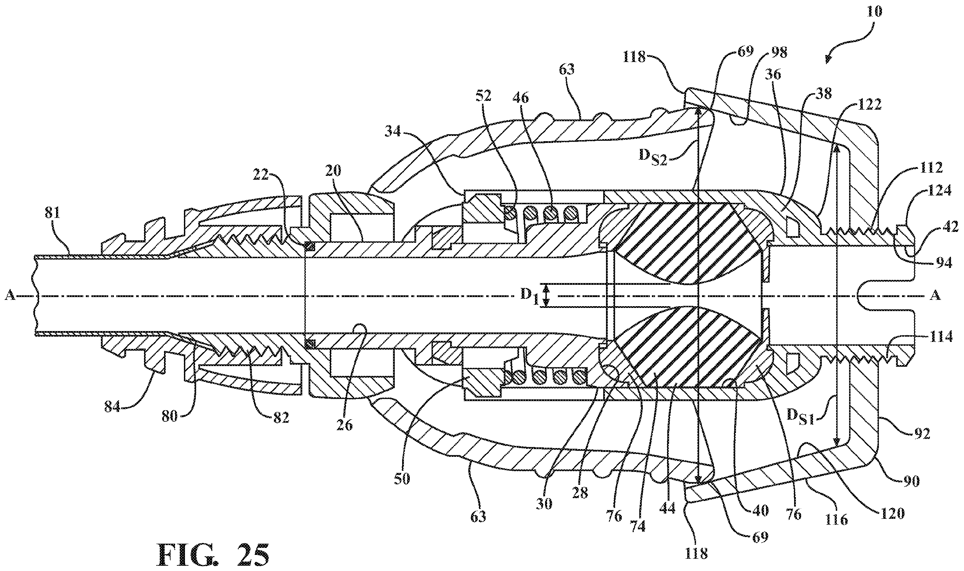

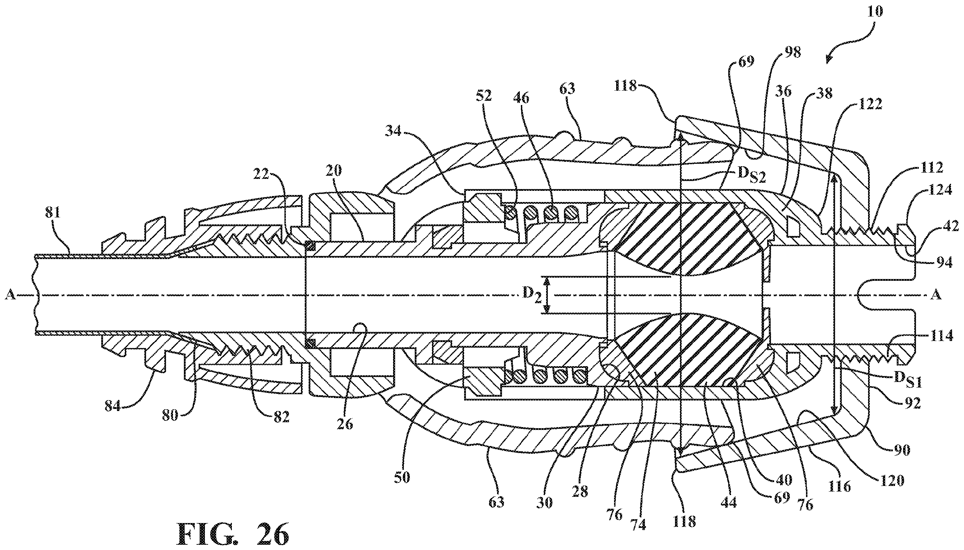

As best shown in Figure 7, a detachable cap **72** can be snapped or disposed over the second valve housing end **36** of the valve housing **32** to hold the pair of lever arms **63** in the radially compressed position when the medical valve assembly **10** is not in use. When the detachable cap **72** is in place, it keeps the elastomeric seal **44** in the open position, and thus increases the shelf life by reducing material creep, material sticking, and/or the distorting of the elastomeric seal **44.** However, as best shown in Figures 11-27, in an alternative arrangement a locking ring **90** can be rotatably connected to the second valve housing end **36** and rotatable relative to the pair of lever arms **63** to establish and maintain a radially compressed condition of the pair of lever arms **63** and an increased inner diameter **46** of the elastomeric seal **44.** Similar to the detachable cap **72,** the locking ring **90** can also be set to advantageously hold the pair of lever arms **63** in the radially compressed position without user intervention when the medical valve assembly **10** is not in use to keep the elastomeric seal **44** in the open position, and thus increase the shelf life by reducing material creep, material sticking, and/or the distorting of the elastomeric seal **44.** Additionally, as will be explained in more detail below, the locking ring **90** also allows a user of the medical valve assembly **10** to place and maintain the elastomeric seal in the open condition, or a plurality of other positions, during use of the medical valve assembly **10** for allowing a user to insert a medical device **12** therethrough without the need for the user to constantly maintain a manual radial compression on the pair of lever arms. Thus, the locking ring **90** also improves the user experience during use of the medical valve assembly **10.**

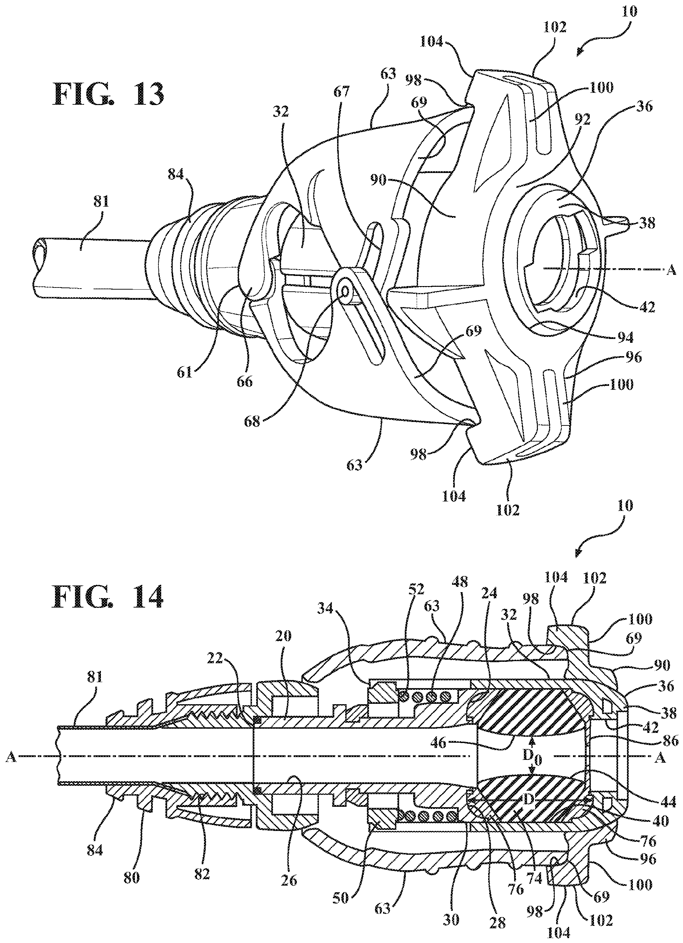

The locking ring **90** is rotatable from a first, closed position (as best shown in Figures 11-12, 15, and 24), wherein the locking ring **90** is disposed in spaced and non-engaging relationship with the pair of lever arms **63** to establish the closed condition of the elastomeric seal **44,** to a second, open position (as best shown in Figures 13-14, 21-22B, and 26) wherein the locking ring **90** engages and completely or fully radially compresses the pair of lever arms **63** to establish and maintain an increased and open inner diameter **D₀** of the elastomeric seal **44.** As best shown in Figures 14, 22A, 22B, and 27, the open inner diameter **D₀** of the elastomeric seal **44,** as established and maintained by the locking ring **90,** establishes a respective open condition of the medical valve assembly **10** for allowing a medical device **12** to pass therethrough.

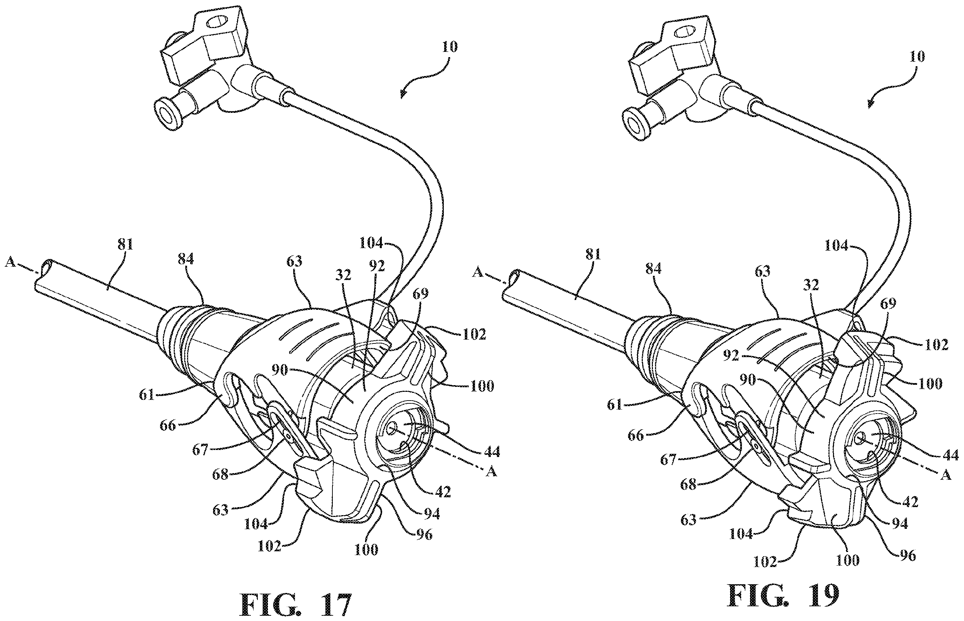

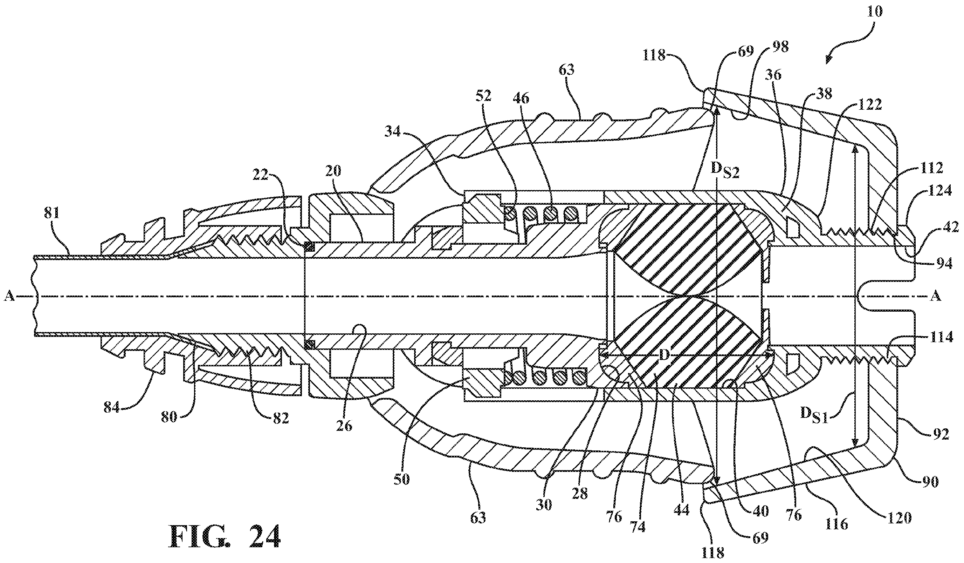

As further illustrated in Figures 17-27, in an additional arrangement, the locking ring **90** is also rotatable to at least one intermediate position disposed between the open and closed positions to partially radially compress the pair of lever arms **63** and establish at least one intermediate inner diameter of the elastomeric seal **44** that is greater than the closed inner diameter of the elastomeric seal **44** (which as illustrated in Figures 12 and 24 is preferably zero since the elastomeric seal **44** is compressed upon itself) but less than the open inner diameter **D₀** of the elastomeric seal **44** illustrated in Figures 14, 22A, 22B, and 27. As previously discussed, the medical valve assembly **10** must be able to provide a hemostatic seal under a variety of conditions, i.e., accommodate a variety of different sized medical devices **12.** Thus, the locking ring **90** including at least one intermediate position advantageously allows a user to quickly and incrementally adjust an inner diameter of the elastomeric seal **44** to accommodate use of differently sized medical devices. In other words, each intermediate position as well as the open position would corresponding to a desired amount of opening of the elastomeric seal **44** of the medical valve **10.** For example, a medical device **12** with a larger sized diameter could be utilized when the locking ring **90** is disposed in the open condition while a different medical device with a relatively smaller sized diameter could be utilized when the locking ring **90** is disposed in the at least one intermediate position. The locking ring **90** which incorporates at least one intermediate position also allows the user to place and maintain the elastomeric seal **44** in each of the open and the at least one intermediate positions without the need for the user to continually and constantly maintain a manual radial compression on the pair of lever arms to maintain the appropriate size for allowing insertion of the differently sized devices. Thus, the at least one intermediate position of the locking ring **90** further improves on the user experience during use of the medical valve assembly **10.**

As shown in Figures 17-18B and 25, in a preferred arrangement the at least one intermediate position includes a first intermediate position disposed between the open and closed positions to incrementally radially compress the pair of lever arms **63** relative to the immediately preceding closed condition and establish a first intermediate inner diameter **D₁** being greater than the closed (i.e., fully compressed) inner diameter of the elastomeric seal **44.** As best shown in Figures 19-20B and 25, in a preferred arrangement the at least one intermediate position also includes a second intermediate position disposed between the first intermediate position (Figures 17-18B and 25) and the second, open position (Figures 14, 22A, 22B, and 27) to incrementally radially compress the pair of lever arms **63** relative to the first intermediate position and establish a second intermediate inner diameter **D₂** that is greater than the first intermediate inner diameter **D₁** but less than the open inner diameter **D₀**. The locking ring **90** which incorporates more than one intermediate position allows the locking ring **90** to establish and maintain at least three incrementally sized inner diameters **D₁, D₂, D₀** of the elastomeric seal **44** for advantageously accommodating a large variety of differently sized medical devices **12.** While three different positions are illustrated and described (two intermediate positions plus one open position), it should be appreciated that additional or less intermediate positions can be utilized without departing from the scope of the subject disclosure, namely because the user requires fewer or more positions to accommodate their differently sized medical devices.

As further illustrated in Figures 11-27, the locking ring **90** includes a base **92** rotatably connected to the second valve housing end **36** and defining a base orifice **94** axially aligned with the opening **42** defined by the housing flange **38.** The locking ring **90** includes a locking element **96** extending from the base **92** and defining a locking surface **98** disposed in engaged and sliding relationship with the arcuate second lever arm end **36** during rotation of the locking ring **90** between the first and second positions. In other words, the locking surface **98** is configured to slide along the arcuate shape of the second lever arm ends **36** and effectuate a radial compression of the pair of lever arms **63** during rotation.

As best illustrated in Figures 11-22B, in a first arrangement of the locking ring **90,** the locking element **96** includes a pair of wings **100** disposed in diametrically opposed relationship to one another and each extending from the base **92** to a wing end **102.** In this arrangement, a pair of locking flanges **104** each extend axially towards the first valve housing end **34** from a respective one of the wing ends **102** to define the locking surface **98** disposed on each of the wings **100.** Each of the locking surfaces **98** concurrently interact with and slide along a respective one of the pair of lever arms **63** during rotation of the locking ring **90** to establish any of the previously mentioned positions.

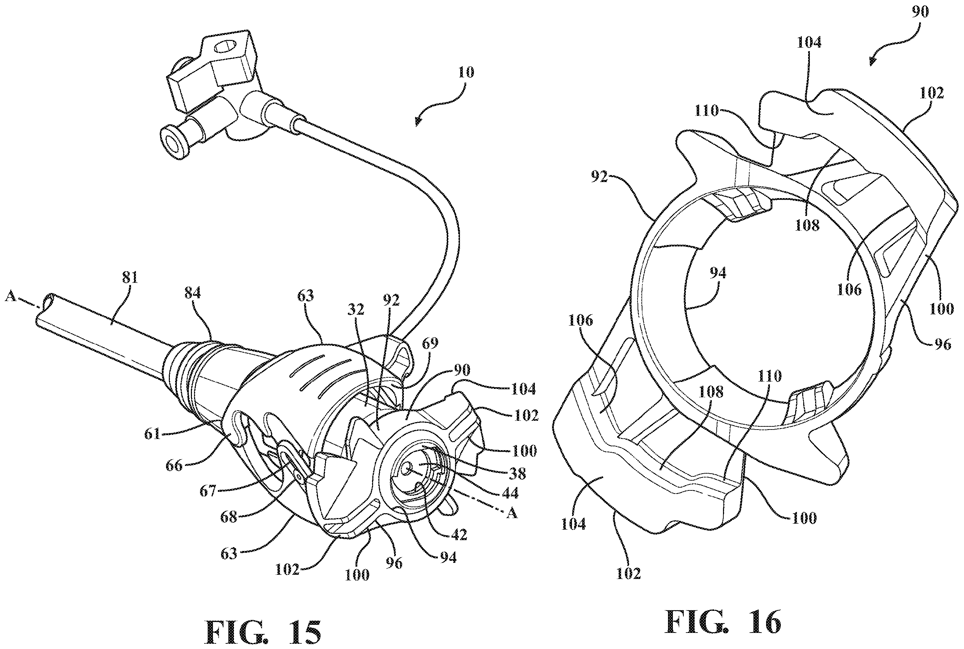

As best illustrated in Figure 16, 18B, 20B, and 22B, each of the locking surfaces **98** in the first arrangement of the locking element **96** can present a plurality of steps **106, 108, 110** to incrementally and consecutively radially compress the pair of lever arms **63** and establish and maintain the intermediate positions during rotation of the locking ring **90** between the closed and open positions. For example, as best illustrated in Figures 16-18B, the plurality of steps **106, 108, 110** can include a first step **106** to initially engage a respective one of the second lever arm ends **69** of the pair of lever arms **63** and establish the first intermediate position during initial rotation of the locking ring **90** from the first, closed position. As previously discussed, the first intermediate position effectuates a first incremental increase of the distance between the plunger plate **28** and the housing flange **38** and maintains a first intermediate inner diameter **D₁** of the elastomeric seal **44** which is greater than the closed inner diameter (i.e. compressed and preferably zero) but less than the open inner diameter **D₀** of said elastomeric seal **44.** As further illustrated in Figures 16 and 19-20B, the plurality of steps **106, 108, 110** can also include a second step **108** to sequentially engage the respective one of the second lever arm ends **69** of the pair of lever arms **63** and establish the second intermediate position during sequential rotation of the locking ring **90** from the first intermediate position. As previously discussed, the second intermediate position effectuates a second incremental increase of the distance between the plunger plate **28** and the housing flange **38** and maintains a second intermediate inner diameter **D₂** of the elastomeric seal **44** being greater than the first intermediate inner diameter **D₁** but less than the open inner diameter **Do.** As best illustrated in Figures 16 and 21-22B, the plurality of steps **106, 108, 110** can also include a third step **110** to sequentially engage the respective one of the second lever arm ends **69** of the pair of lever arms **63** for effectuating a third incremental increase of the distance between the plunger plate **28** and the housing flange **38** to finally establish the open position and maintain the open inner diameter **D₀** of the elastomeric seal **44.** As previously mentioned, additional steps could be added to the locking ring **90** to incorporate additional incremental increases of the inner diameter as required by the user.

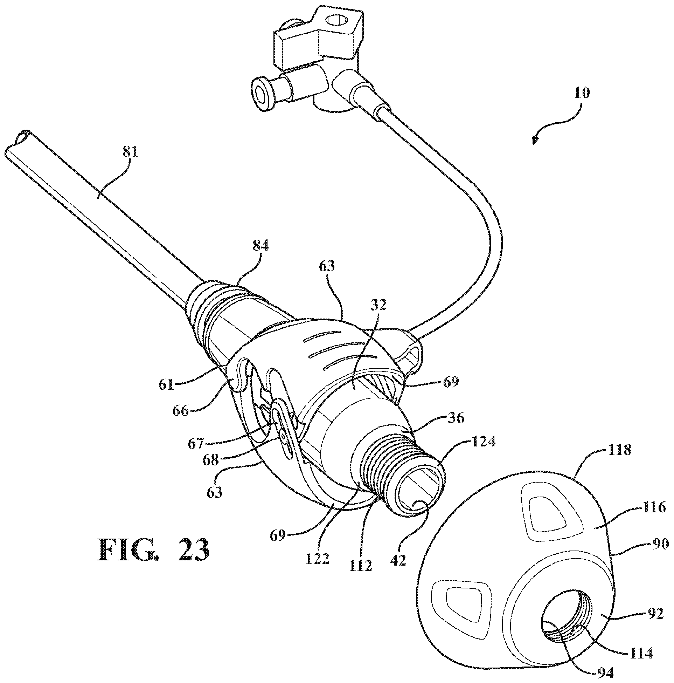

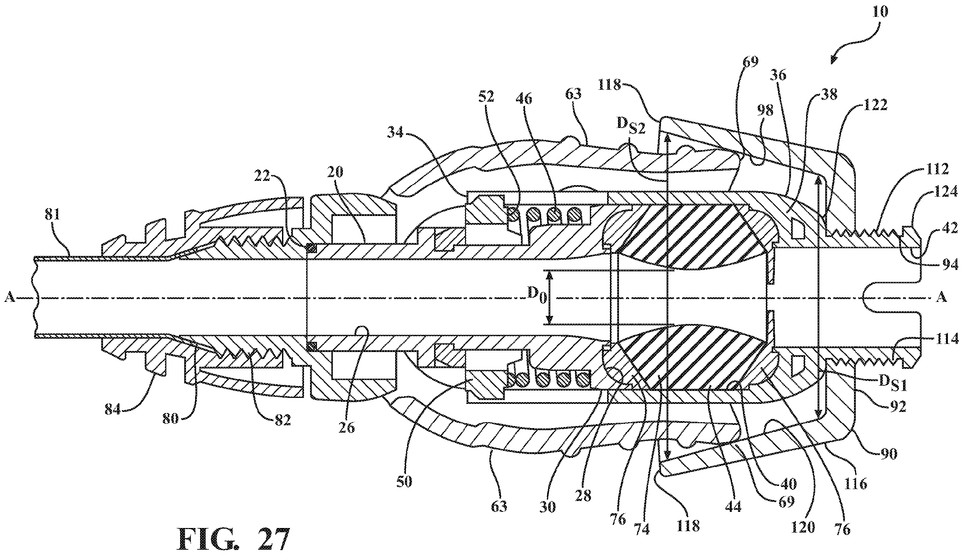

As best illustrated in Figure 23, in another arrangement of the locking ring **90,** the valve housing **32** defines valve threads **112** disposed adjacent the second valve housing end **36.** The base **92** of the locking ring **90** correspondingly defines locking threads **114** extending concentrically around the base orifice **94** and threadingly engaged with the valve threads **112** to establish the rotatable connection between the locking ring **90** and the valve housing **32.** In this arrangement, the locking element **96** includes a shroud **116** extending concentrically around the axis **A** and extending between the base **92** and a shroud end **118** to define a shroud inner surface **120** that establishes the locking surface **98.** As best illustrated in Figures 24-27, the locking ring **90** is rotatable about the valve threads **112** to axially advance the locking ring **90** from the first, closed position (as shown in Figure 24) wherein the shroud end **118** is disposed in spaced relationship with the second lever arm ends **69** of the pair of lever arms **62** to the second, open position (as shown in Figure 27) wherein the shroud inner surface **120** effectuates a full and complete radially compression of the pair of lever arms **63.** The shroud **120** has a first shroud diameter **D_{S1}** disposed and extending adjacent the base **92** and a second shroud diameter **D_{S2}** disposed and extending adjacent the shroud end **118,** with the second shroud diameter **D_{S2}** sized greater than the first shroud diameter **D_{S1}** to define a ramped or tapered locking surface **98.** As shown in Figures 24-27, the ramped locking surface **98** incrementally and sequentially radially compresses the pair of lever arms **63** during movement of the locking ring **90** from the first, closed position (Figure 24) to the second, open position (Figure 27). In other words, as the locking ring **90** is axially advanced along the valve threads **112,** the ramped locking surface **98** initially engages the second lever arm ends **69** and radially compresses the pair of lever arms **63** as the shroud inner diameter narrows from the second shroud diameter **D_{S2}** towards the first inner shroud diameter **D_{S1}**.

As will be appreciated from the aforementioned disclosure, the ramped locking surface **98** establishes a plurality of intermediate positions as the locking ring **90** is axially advanced along the valve threads **112** that can be tailored by the user anywhere between the first, closed position and the second, open position. In other words, since each of the plurality of intermediate positions are established by rotating the locking ring **90** to different axial positions along the valve threads **112,** each different axial location effectuates an incremental and sequential increase of the inner diameter of the elastomeric seal 44, with each intermediate inner diameter of the elastomeric seal **44** being greater than a preceding one of the plurality of axial intermediate positions and less than a subsequent one of said plurality of axial intermediate positions along the valve threads **112.** Thus, a user of the medical valve assembly **10** is provided with increased flexibility to establish an exact size of the inner diameter that is specifically sized to the corresponding medical device **12** for insertion through the medical valve assembly **10.** Once this preferred inner diameter size is established by the user, the locking ring **90** can be left in its axial position along the valve threads **112** to maintain this desired inner diameter size without requiring the user to manually maintain compression of the pair of lever arms **63.** Thus, the shroud of the locking ring **90** further improves on the user experience during use of the medical valve assembly **10.**

As best illustrated in Figures 24-27, the second valve housing end **36** defines a stop **122** and the valve threads **112** extend from the stop **122** and terminate at a lip **124** defined by the valve housing **32.** Thus, as best illustrated in Figure 24, the base **92** of the locking ring **90** is disposed in abutting relationship with the lip **124** in the first, closed position and, as best illustrated in Figure 27, is axially advanced into abutting relationship with the stop **122** to establish the second, open position.

In any arrangement of the medical valve assembly **10,** the elastomeric seal **44** can include an inner portion **74** and an outer portion **76** disposed axially outwardly from the inner portion **74.** In a preferred embodiment, the inner portion **74** is made from a first material having a first durometer value and the outer portion **76** is made from a second material having a second durometer value being greater than the first durometer value. In other words, the elastomeric seal **44** includes an outside portion **76** that is harder than an inside portion **74.** As further shown in Figures 4A and 4B, the outer portion **76** of the elastomeric seal **44** is disposed in compressed relationship with the plunger plate **28** and the housing flange **38.** In a preferred embodiment, the plunger plate **28** and the housing flange **38** can include curved portions **78** to improve the retention and compression of the outer portions **76** of the elastomeric seal **44.**

As best shown in Figure 4, the tube **20** has a tapered portion **80** disposed adjacent the first tube end **22** for fitting a sheath **81** over the tube **20.** The tube **20** includes nose cap threads **82** disposed adjacent the first tube end **22** and a nose cap **84** is threadingly secured to the first tube end **22** for establishing a compression fit of the sheath between the nose cap **84** and the tapered portion **80** of the tube **20.** Although not expressly shown, a wiper seal can be disposed within the passageway **26** between the elastomeric seal **44** and the first tube end **22** to provide a level of hemostasis around a larger device while the elastomeric seal **44** is opened for insertion of the medical device **12.** Alternatively, as best shown in Figures 5, 12, 14, 18, 20, 22, and 24-26, a wiper seal **86** can be incorporated into the elastomeric seal **44** and extends radially inward from one of the outside portions **76.** As a result, when the elastomeric seal **44** is inserted in the cavity **40,** the wiper seal **86** is disposed adjacent the opening **42** of the valve housing **32.**

It will be appreciated by those skilled in the art that the medical valve assembly **10'** shown in Figures 8 and 9 can be equipped with the compression type manual actuator **62** shown in Figures 3, 4 and 7 or, in the alternative, the pull-type manual actuator **60** shown in Figure 6. Likewise, alternative configurations are contemplated for manual actuators that function to controllably vary the relative axial position between two components for proportionately controlling the compression load applied to an elastomeric seal to regulate an internal opening dimension defined thereby.

The foregoing description of the embodiments has been provided for purposes of illustration and description. It is not intended to be exhaustive or to limit the disclosure.

## Claims

1. A medical valve assembly (10) for use in inserting a medical device (12) into a body vessel (14) of a patient (16), comprising:
a tube (20) extending between a first tube end (22) and a second tube end (24) to define a passageway (26) extending along a longitudinal axis (A) between said ends (22, 24);
a plunger plate (28) extending radially from said second tube end (24) of said tube (20);
a valve housing (32) surrounding said tube (20) about said second tube end (24) and extending from a first valve housing end (34) to a second valve housing end (36);
said valve housing (32) including a housing flange (38) extending radially inwards from said second valve housing end (36) and disposed in spaced relationship with said plunger plate (28) to define a distance (D) extending therebetween;
an elastomeric seal (44) compressed between said plunger plate (28) and said housing flange (38) and having an inner diameter (46) for establishing a variable seal of the medical valve assembly (10);
a compression member (48) disposed within said valve housing (32) and biased against said plunger plate (28) for decreasing said inner diameter (46) to establish a closed inner diameter of the elastomeric seal (44) and a respective closed condition of the medical valve assembly;
a pair of lever arms (63) pivotably connected to said valve housing (32) and radially compressible to overcome the bias of said compression member (48) and effectuate axial movement of said valve housing (32) relative to said tube (20) to increase the distance (D) between said plunger plate (28) and said housing flange (32) and increase said inner diameter (46) of said elastomeric seal (44) from the closed condition; and
a locking ring (90) rotatably connected to said second valve housing end (36) and rotatable relative to said pair of lever arms (63) from a closed position wherein said locking ring (90) is disposed in spaced and non-engaging relationship with said pair of lever arms (63) to establish said closed condition of the medical valve assembly (10) to an open position wherein said locking ring (90) engages and radially compresses said pair of lever arms (63) to establish and maintain an increased open inner diameter (Dₒ) of said elastomeric seal (44) and a respective open condition of said elastomeric seal (44); and
wherein said locking ring (90) is rotatable to at least one intermediate position disposed between said open and closed positions to partially radially compress said pair of lever arms (63) and establish at least one intermediate inner diameter (D₁, D₂) of said elastomeric seal (44) being greater than said closed inner diameter of said elastomeric seal (44) but less than said open inner diameter (Dₒ) of said elastomeric seal (44).

2. A medical valve assembly (10) as set forth in Claim 1, wherein said at least one intermediate position includes a first intermediate position disposed between said open and closed positions to incrementally radially compress said pair of lever arms (63) relative to said closed condition and establish a first intermediate inner diameter (D₁) being greater than said closed inner diameter of said elastomeric seal (44), and wherein said at least one intermediate position includes a second intermediate position disposed between said first intermediate position and said open position to incrementally radially compress said pair of lever arms (63) relative to said first intermediate position and establish a second intermediate inner diameter (D₂) being greater than said first intermediate inner diameter (D₁) but less than said open inner diameter (Dₒ).

3. A medical valve assembly (10) as set forth in Claim 1, wherein said housing flange (32) defines an opening (42) aligned on said axis (A) and sized to receive the medical device, and wherein said locking ring (90) includes a base (92) rotatably connected to said second valve housing end (36) and defining a base orifice (94) axially aligned with said opening (42).

4. A medical valve assembly (10) as set forth in Claim 3, wherein each of said lever arms (63) extend from a first lever arm end (61) pivotably connected to said tube (20) to a second lever arm end (69) disposed adjacent said second valve housing end (36) and having an arcuate shape relative to said axis (A), and wherein said locking ring (90) includes a locking element (96) extending from said base (92) and defining a locking surface (98) disposed in engaged and sliding relationship with said arcuate second lever arm end (69) during rotation of said locking ring (90) between said closed and open positions.

5. A medical valve assembly (10) as set forth in Claim 4, wherein said locking element (96) includes a pair of wings (100) disposed in diametrically opposed relationship to one another and each extending from said base (92) to a wing end (102), and a pair of locking flanges (104) each extending axially towards said first valve housing end (34) from a respective one of said wing ends (102) to define said locking surface (98) disposed on each of said wings (100) for establishing and maintain said increased open inner diameter (Dₒ) of said elastomeric seal (44) when said locking ring (90) is disposed in said open position.

6. A medical valve assembly (10) as set forth in Claim 5, wherein each of said locking surfaces (98) presents a plurality of steps (106, 108, 110) to incrementally and sequentially radially compress said pair of lever arms (63) and establish and maintain said at least one intermediate position during rotation of said locking ring (90) from said closed position to said open position for maintaining said at least one intermediate inner diameter (D₁, D₂) of said elastomeric seal (44).

7. A medical valve assembly (10) as set forth in claim 6, further comprising:
wherein said plurality of steps (106, 108, 110) includes a first step (106) to initially engage a respective one of said second lever arm ends (69) of said pair of lever arms (63) and establish a first intermediate position during initial rotation of said locking ring (90) from said closed position, wherein said first intermediate position effectuates and maintains a first intermediate inner diameter (D₁) of said elastomeric seal (44) being greater than said closed inner diameter of said elastomeric seal (44) but less than said open inner diameter (D₀) of said elastomeric seal (44);
wherein said plurality of steps (106, 108, 110) includes a second step (108) to sequentially engage said respective one of said second lever arm ends (69) of said pair of lever arms (63) and establish a second intermediate position during sequential rotation of said locking ring (90) from said first intermediate position, wherein said second intermediate position effectuates and maintains a second intermediate inner diameter (D₂) of said elastomeric seal (44) being greater than said first intermediate inner diameter (D₁) but less than said open inner diameter (D₀) of said elastomeric seal (44); and
wherein said plurality of steps (106, 108, 110) includes a third step (110) to sequentially engage said respective one of said second lever arm ends (69) of said pair of lever arms (63) for effectuating a third incremental increase of the distance D between said plunger plate (28) and said housing flange (38) to establish said open position and maintain said open inner diameter (D₀) of said elastomeric seal (44).

8. A medical valve assembly (10) as set forth in Claim 3, further comprising:
said valve housing (32) defining valve threads (112) disposed adjacent said second valve housing end (36); and
said base (92) of said locking ring (90) defining locking threads (114) extending concentrically around said base orifice (96) and threadingly engaged with said valve threads (112) to establish said rotatable connection between said locking ring (90) and said valve housing (32).

9. A medical valve assembly (10) as set forth in Claim 8, wherein said locking element (96) includes a shroud (116) extending concentrically around said axis (A) and extending from said base (92) to a shroud end (118) to define a shroud inner surface (120) for establishing said locking surface (98), and wherein said locking ring (90) is rotatable about said valve threads (114) to axially advance said locking ring (90) from said closed position wherein said shroud end (118) is disposed in spaced relationship with said second lever arm ends (69) of said pair of lever arms (63) to said open position wherein said shroud inner surface (120) radially compresses said pair of lever arms (63).

10. A medical valve assembly (10) as set forth in Claim 9, wherein said shroud (116) having a first shroud diameter (D_{S1}) adjacent said base (92) and a second shroud diameter (D_{S2}) adjacent said shroud end (118), said second shroud diameter (D_{S2}) being greater than said first shroud diameter (D_{S1}) to define a ramped locking surface (98) for incrementally and sequentially radially compressing said pair of lever arms (63) during movement of said locking ring (90) from said closed position to said open position.

11. A medical valve assembly (10) as set forth in Claim 10, wherein said ramped locking surface (98) establishes a plurality of intermediate positions during axial movement of said locking ring (90) from said closed position to said open position, and wherein each of said plurality of intermediate positions effectuate an incremental and sequential increase of said distance (D) between said plunger plate (28) and said housing flange (38) and maintains a respective intermediate inner diameter of said elastomeric seal (44) being greater than a preceding one of said plurality of intermediate positions and less than a subsequent one of said plurality of intermediate positions.

12. A medical valve assembly (10) as set forth in Claim 11, wherein said second valve housing end (36) defines a stop (122) and said valve threads (112) extend from said stop (122) and terminate at a lip (124), and wherein said base (92) of said locking ring (90) is disposed in abutting relationship with said lip (124) in said closed position and is axially advanced into abutting relationship with said stop (122) to establish said open position.

## Patentansprüche

1. Medizinische Ventilanordnung (10) zur Verwendung beim Einführen einer medizinischen Vorrichtung (12) in ein Körpergefäß (14) eines Patienten (16), umfassend:
ein Rohr (20), das sich zwischen einem ersten Rohrende (22) und einem zweiten Rohrende (24) erstreckt, um einen Durchgang (26) zu definieren, der sich entlang einer Längsachse (A) zwischen den Enden (22, 24) erstreckt;
eine Plungerplatte (28), die sich radial von dem zweiten Rohrende (24) des Rohrs (20) erstreckt;
ein Ventilgehäuse (32), das das Rohr (20) um das zweite Rohrende (24) herum umgibt und sich von einem ersten Ventilgehäuseende (34) zu einem zweiten Ventilgehäuseende (36) erstreckt;
wobei das Ventilgehäuse (32) einen Gehäuseflansch (38) aufweist, der sich von dem zweiten Ventilgehäuseende (36) radial nach innen erstreckt und in beabstandeter Beziehung zu der Plungerplatte (28) angeordnet ist, um einen sich dazwischen erstreckenden Abstand (D) zu definieren;
eine Elastomerdichtung (44), die zwischen der Plungerplatte (28) und dem Gehäuseflansch (38) komprimiert wird und einen Innendurchmesser (46) aufweist, um eine variable Dichtung der medizinischen Ventilanordnung (10) herzustellen;
ein Kompressionselement (48), das innerhalb des Ventilgehäuses (32) angeordnet und gegen die Plungerplatte (28) vorgespannt ist, um den Innendurchmesser (46) zu verringern, um einen geschlossenen Innendurchmesser der Elastomerdichtung (44) und einen entsprechenden geschlossenen Zustand der medizinischen Ventilanordnung herzustellen;
ein Paar von Hebelarmen (63), die schwenkbar mit dem Ventilgehäuse (32) verbunden und radial komprimierbar sind, um die Vorspannung des Kompressionselements (48) zu überwinden und eine axiale Bewegung des Ventilgehäuses (32) relativ zu dem Rohr (20) zu bewirken, um aus dem geschlossenen Zustand den Abstand (D) zwischen der Plungerplatte (28) und dem Gehäuseflansch (32) zu vergrößern und den Innendurchmesser (46) der Elastomerdichtung (44) zu vergrößern; und
einen Verriegelungsring (90), der drehbar mit dem zweiten Ventilgehäuseende (36) verbunden und relativ zu dem Paar von Hebelarmen (63) aus einer geschlossenen Position, in der der Verriegelungsring (90) in einer beabstandeten und nicht in Eingriff stehenden Beziehung mit dem Paar von Hebelarmen (63) angeordnet ist, um den geschlossenen Zustand der medizinischen Ventilanordnung (10) herzustellen, in eine offene Position drehbar ist, in der der Verriegelungsring (90) mit dem Paar von Hebelarmen (63) in Eingriff steht und diese radial komprimiert, um einen vergrößerten offenen Innendurchmesser (D₀) der Elastomerdichtung (44) und einen entsprechenden offenen Zustand der Elastomerdichtung (44) herzustellen und aufrecht zu erhalten; und
wobei der Verriegelungsring (90) in mindestens eine Zwischenposition drehbar ist, die zwischen der offenen und der geschlossenen Position angeordnet ist, um das Paar von Hebelarmen (63) teilweise radial komprimieren und mindestens einen Zwischen-Innendurchmesser (D₁, D₂) der Elastomerdichtung (44) herzustellen, der größer als der geschlossene Innendurchmesser der Elastomerdichtung (44), aber kleiner als der offene Innendurchmesser (D₀) der Elastomerdichtung (44) ist.

2. Medizinische Ventilanordnung (10) nach Anspruch 1, wobei die mindestens eine Zwischenposition eine erste Zwischenposition umfasst, die zwischen der offenen und der geschlossenen Position angeordnet ist, um das Paar von Hebelarmen (63) relativ zu dem geschlossenen Zustand inkrementell radial komprimieren und einen ersten Zwischen-Innendurchmesser (D₁) herzustellen, der größer ist als der geschlossene Innendurchmesser der Elastomerdichtung (44), und wobei die mindestens eine Zwischenposition eine zweite Zwischenposition umfasst, die zwischen der ersten Zwischenposition und der offenen Position angeordnet ist, um das Paar von Hebelarmen (63) relativ zu der ersten Zwischenposition inkrementell radial komprimieren und einen zweiten Zwischen-Innendurchmesser (D₂) herzustellen, der größer ist als der erste Zwischen-Innendurchmesser (D₁₎, aber kleiner als der offene Innendurchmesser (D₀).

3. Medizinische Ventilanordnung (10) nach Anspruch 1, wobei der Gehäuseflansch (32) eine Öffnung (42) definiert, die auf der Achse (A) ausgerichtet und so bemessen ist, dass sie die medizinische Vorrichtung aufnimmt, und wobei der Verriegelungsring (90) eine Basis (92) umfasst, die drehbar mit dem zweiten Ventilgehäuseende (36) verbunden ist und eine Basisöffnung (94) definiert, die axial mit der Öffnung (42) ausgerichtet ist.

4. Medizinische Ventilanordnung (10) nach Anspruch 3, wobei sich jeder der Hebelarme (63) von einem ersten Hebelarmende (61), das schwenkbar mit dem Rohr (20) verbunden ist, zu einem zweiten Hebelarmende (69) erstreckt, das benachbart zu dem zweiten Ventilgehäuseende (36) angeordnet ist und eine bogenförmige Gestalt relativ zu der Achse (A) aufweist, und wobei der Verriegelungsring (90) ein Verriegelungselement (96) aufweist, das sich von der Basis (92) aus erstreckt und eine Verriegelungsfläche (98) definiert, die während der Drehung des Verriegelungsrings (90) zwischen der geschlossenen und der offenen Stellung in Eingriff und in Gleitbeziehung mit dem bogenförmigen zweiten Hebelarmende (69) angeordnet ist.

5. Medizinische Ventilanordnung (10) nach Anspruch 4, wobei das Verriegelungselement (96) ein Paar von Flügeln (100), die in diametral gegenüberliegender Beziehung zueinander angeordnet sind und sich jeweils von der Basis (92) zu einem Flügelende (102) erstrecken, und ein Paar von Verriegelungsflanschen (104) umfasst, die sich jeweils axial in Richtung des ersten Ventilgehäuseendes (34) von einem jeweiligen der Flügelenden (102) aus erstrecken, um die Verriegelungsfläche (98) zu definieren, die auf jedem der Flügel (100) angeordnet ist, um den vergrößerten offenen Innendurchmesser (D₀) der Elastomerdichtung (44) herzustellen und beizubehalten, wenn der Verriegelungsring (90) in der offenen Position angeordnet ist.

6. Medizinische Ventilanordnung (10) nach Anspruch 5, wobei jede der Verriegelungsflächen (98) mehrere Schritte (106, 108, 110) aufweist, um das Paar von Hebelarmen (63) inkrementell und sequentiell radial komprimieren und die mindestens eine Zwischenposition während der Drehung des Verriegelungsrings (90) von der geschlossenen Position in die offene Position einzurichten und beizubehalten, um den mindestens einen inneren Zwischendurchmesser (D₁, D₂) der Elastomerdichtung (44) beizubehalten.

7. Medizinische Ventilanordnung (10) nach Anspruch 6, ferner umfassend:
wobei die mehreren Schritte (106, 108, 110) einen ersten Schritt (106) umfassen, um anfänglich mit einem jeweiligen der zweiten Hebelarmenden (69) des Paars von Hebelarmen (63) in Eingriff zu kommen und eine erste Zwischenposition während der anfänglichen Drehung des Verriegelungsrings (90) aus der geschlossenen Position einzurichten, wobei die erste Zwischenposition einen ersten Zwischeninnendurchmesser (D₁) der Elastomerdichtung (44) bewirkt und aufrechterhält, der größer als der geschlossene Innendurchmesser der Elastomerdichtung (44), aber kleiner als der offene Innendurchmesser (D₀) der Elastomerdichtung (44) ist;
wobei die mehreren Schritte (106, 108, 110) einen zweiten Schritt (108) umfassen, um nacheinander mit dem jeweiligen einen der zweiten Hebelarmenden (69) des Paars von Hebelarmen (63) in Eingriff zu kommen und während der sequentiellen Drehung des Verriegelungsrings (90) aus der ersten Zwischenposition eine zweite Zwischenposition einzurichten, wobei die zweite Zwischenposition einen zweiten Zwischeninnendurchmesser (D₂) der Elastomerdichtung (44) bewirkt und aufrechterhält, der größer als der erste Zwischeninnendurchmesser (D₁), aber kleiner als der offene Innendurchmesser (D₀) der Elastomerdichtung (44) ist; und
wobei die mehreren Schritte (106, 108, 110) einen dritten Schritt (110) umfassen, um nacheinander mit dem jeweiligen einen der zweiten Hebelarmenden (69) des Paars von Hebelarmen (63) in Eingriff zu kommen, um eine dritte inkrementelle Vergrößerung des Abstands D zwischen der Plungerplatte (28) und dem Gehäuseflansch (38) zu bewirken, um die offene Position einzurichten und den offenen Innendurchmesser (D₀) der Elastomerdichtung (44) beizubehalten.

8. Medizinische Ventilanordnung (10) nach Anspruch 3, ferner umfassend:
wobei das Ventilgehäuse (32) ein Ventilgewinde (112) aufweist, das benachbart dem zweiten Ventilgehäuseende (36) angeordnet ist; und
wobei die Basis (92) des Verriegelungsrings (90) ein Verriegelungsgewinde (114) definiert, das sich konzentrisch um die Basisöffnung (96) herum erstreckt und in Gewindeeingriff mit dem Ventilgewinde (112) steht, um die drehbare Verbindung zwischen dem Verriegelungsring (90) und dem Ventilgehäuse (32) herzustellen.

9. Medizinische Ventilanordnung (10) nach Anspruch 8, wobei das Verriegelungselement (96) eine Abdeckung (116) aufweist, die sich konzentrisch um die Achse (A) erstreckt und sich von der Basis (92) zu einem Abdeckungsende (118) erstreckt, um eine Abdeckungsinnenfläche (120) zum Bilden der Verriegelungsfläche (98) zu definieren, und wobei der Verriegelungsring (90) um das Ventilgewinde (114) drehbar ist, um den Verriegelungsring (90) von der geschlossenen Position, in der das Abdeckungsende (118) in beabstandeter Beziehung zu den zweiten Hebelarmenden (69) des Paares von Hebelarmen (63) angeordnet ist, in die offene Position vorzuschieben, in der die Abdeckungsinnenfläche (120) das Paar von Hebelarmen (63) radial komprimiert.

10. Medizinische Ventilanordnung (10) nach Anspruch 9, wobei die Abdeckung (116) einen ersten Abdeckungsdurchmesser (D_{S1}) benachbart der Basis (92) und einen zweiten Abdeckungsdurchmesser (D_{S2}) benachbart des Abdeckungsendes (118) aufweist, wobei der zweite Abdeckungsdurchmesser (D_{S2}) größer als der erste Abdeckungsdurchmesser (D_{S1}) ist, um eine Rampenverriegelungsfläche (98) zum inkrementellen und sequentiellen radialen Komprimieren des Paares von Hebelarmen (63) während der Bewegung des Verriegelungsrings (90) von der geschlossenen Position zur offenen Position zu definieren.

11. Medizinische Ventilanordnung (10) nach Anspruch 10, wobei die rampenförmige Verriegelungsfläche (98) während der axialen Bewegung des Verriegelungsrings (90) aus der geschlossenen Position in die offene Position eine Vielzahl von Zwischenpositionen einnimmt, und wobei jede der Vielzahl von Zwischenpositionen eine inkrementelle und sequentielle Vergrößerung des Abstands (D) zwischen der Plungerplatte (28) und dem Gehäuseflansch (38) bewirkt und einen jeweiligen Zwischen-Innendurchmesser der Elastomerdichtung (44) aufrechterhält, der größer als eine vorhergehende der Vielzahl von Zwischenpositionen und kleiner als eine nachfolgende der Vielzahl von Zwischenpositionen ist.

12. Medizinische Ventilanordnung (10) nach Anspruch 11, wobei das zweite Ventilgehäuseende (36) einen Anschlag (122) definiert und das Ventilgewinde (112) sich von dem Anschlag (122) aus erstreckt und an einer Lippe (124) endet, und wobei die Basis (92) des Verriegelungsrings (90) in der geschlossenen Position in anliegender Beziehung mit der Lippe (124) angeordnet ist und axial in anliegende Beziehung mit dem Anschlag (122) vorgeschoben wird, um die offene Position herzustellen.

## Revendications

1. Assemblage de valvule médicale (10) pour une utilisation dans une insertion d'un dispositif médical (12) jusque dans un vaisseau corporel (14) d'un patient (16), comprenant :
un tube (20) s'étendant entre une première extrémité de tube (22) et une seconde extrémité de tube (24) pour définir une voie de passage (26) s'étendant le long d'un axe longitudinal (A) entre lesdites extrémités (22, 24) ;
une plaque à piston (28) s'étendant radialement depuis ladite seconde extrémité de tube (24) dudit tube (20) ;
un logement de valvule (32) entourant ledit tube (20) autour de ladite seconde extrémité de tube (24) et s'étendant depuis une première extrémité de logement de valvule (34) jusqu'à une seconde extrémité de logement de valvule (36) ;
ledit logement de valvule (32) incluant une bride de logement (38) s'étendant radialement vers l'intérieur de ladite seconde extrémité de logement de valvule (36) et disposée dans une relation d'espacement avec ladite plaque à piston (28) pour définir une distance (D) s'étendant entre celles-ci ;
un joint d'étanchéité élastomère (44) comprimé entre ladite plaque à piston (28) et ladite bride de logement (38) et ayant un diamètre intérieur (46) pour établir une étanchéité variable de l'assemblage de valvule médicale (10) ;
un élément de compression (48) disposé à l'intérieur dudit logement de valvule (32) et sollicité contre la plaque à piston (28) pour diminuer ledit diamètre intérieur (46) afin d'établir un diamètre intérieur fermé du joint d'étanchéité élastomère (44) et une condition fermée respective de l'assemblage de valvule médicale ;
une paire de bras de levier (63) connectés de manière pivotante audit logement de valvule (32) et pouvant être comprimés radialement pour surmonter la sollicitation dudit élément de compression (48) et effectuer un déplacement axial dudit logement de valvule (32) relativement audit tube (20) pour diminuer la distance (D) entre ladite plaque à piston (28) et ladite bride de logement (32) et pour augmenter ledit diamètre intérieur (46) dudit joint d'étanchéité élastomère (44) depuis la condition fermée ; et
une bague de verrouillage (90) connectée en rotation à ladite seconde extrémité de logement de valvule (36) et pouvant être mise en rotation relativement à ladite paire de bras de levier (63) depuis une position fermée dans laquelle ladite bague de verrouillage (90) est disposée dans une relation d'espacement et de non-engagement avec ladite paire de bras de levier (63) pour établir ladite condition fermée de l'assemblage de valvule médicale (10) jusqu'à une position ouverte dans laquelle ladite bague de verrouillage (90) engage et comprime radialement ladite paire de bras de levier (63) pour établir et maintenir un diamètre intérieur ouvert augmenté (D₀) dudit joint d'étanchéité élastomère (44) et une condition ouverte respective dudit joint d'étanchéité élastomère (44) ; et
dans lequel ladite bague de verrouillage (90) peut être mise en rotation jusqu'à au moins une position intermédiaire disposée entre ladite position ouverte et ladite position fermée pour comprimer radialement en partie ladite paire de bras de levier (63) et établir au moins un diamètre intérieur intermédiaire (D₁, D₂) dudit joint d'étanchéité élastomère (44) qui est supérieur audit diamètre intérieur fermé dudit joint d'étanchéité élastomère (44) mais qui est inférieur audit diamètre intérieur ouvert (D₀) dudit joint d'étanchéité élastomère (44).

2. Assemblage de valvule médicale (10) selon la revendication 1, dans lequel ladite au moins une position intermédiaire inclut une première position intermédiaire disposée entre ladite position ouverte et ladite position fermée pour comprimer radialement de manière incrémentale ladite paire de bras de levier (63) relativement à ladite condition fermée et pour établir un premier diamètre intérieur intermédiaire (D₁) qui est supérieur audit diamètre intérieur fermé dudit joint d'étanchéité élastomère (44), et dans lequel ladite au moins une position intermédiaire inclut une seconde position intermédiaire disposée entre ladite première position intermédiaire et ladite position ouverte pour comprimer radialement de manière incrémentale ladite paire de bras de levier (63) relativement à ladite première position intermédiaire et pour établir un second diamètre intérieur intermédiaire (D₂) qui est supérieur audit premier diamètre intérieur intermédiaire (D₁) mais qui est inférieur audit diamètre intérieur ouvert (D₀).

3. Assemblage de valvule médicale (10) selon la revendication 1, dans lequel ladite bride de logement (32) définit une ouverture (42) alignée sur ledit axe (A) et dimensionnée pour recevoir le dispositif médical, et dans lequel ladite bague de verrouillage (90) inclut une base (92) connectée en rotation à ladite seconde extrémité de logement de valvule (36) et définissant un orifice de base (94) aligné axialement avec ladite ouverture (42).

4. Assemblage de valvule médicale (10) selon la revendication 3, dans lequel chacun desdits bras de levier (63) s'étend depuis une première extrémité de bras de levier (61) connectée de manière pivotante audit tube (20) jusqu'à une seconde extrémité de bras de levier (69) disposée de manière adjacente à ladite seconde extrémité de logement de valvule (36) et ayant une forme arquée relativement audit axe (A), et dans lequel ladite bague de verrouillage (90) inclut un élément de verrouillage (96) s'étendant depuis ladite base (92) et définissant une surface de verrouillage (98) disposée dans une relation d'engagement et de coulissement avec ladite seconde extrémité de levier de bras arquée (69) pendant une rotation de ladite bague de verrouillage (90) entre ladite position ouverte et ladite position fermée.

5. Assemblage de valvule médicale (10) selon la revendication 4, dans lequel ledit élément de verrouillage (96) inclut une paire d'ailes (100) disposées dans une relation diamétralement opposée l'une par rapport à l'autre et s'étendant chacune depuis ladite base (92) jusqu'à une extrémité d'aile (102), et une paire de brides de verrouillage (104) s'étendant chacune axialement vers ladite première extrémité de logement de valvule (34) depuis une extrémité respective desdites extrémités d'aile (102) pour définir ladite surface de verrouillage (98) disposée sur chacune desdites ailes (100) pour établir et maintenir ledit diamètre intérieur ouvert augmenté (D₀) dudit joint d'étanchéité élastomère (44) quand ladite bague de verrouillage (90) est disposée dans ladite position ouverte.

6. Assemblage de valvule médicale (10) selon la revendication 5, dans lequel chacune desdites surfaces de verrouillage (98) présente une pluralité de gradins (106, 108, 110) pour comprimer radialement de manière incrémentale et séquentielle ladite paire de bras de levier (63) et pour établir et maintenir ladite au moins une position intermédiaire pendant une rotation de ladite bague de verrouillage (90) depuis ladite position fermée jusqu'à ladite position ouverte afin de maintenir ledit au moins un diamètre intérieur intermédiaire (D₁, D₂) dudit joint d'étanchéité élastomère (44).

7. Assemblage de valvule médicale (10) selon la revendication 6, comprenant en outre :
dans lequel ladite pluralité de gradins (106, 108, 110) inclut un premier gradin (106) pour engager initialement une extrémité respective desdites secondes extrémités de bras de levier (69) de ladite paire de bras de levier (63) et pour établir une première position intermédiaire pendant une rotation initiale de ladite bague de verrouillage (90) depuis ladite position fermée, dans lequel ladite première position intermédiaire effectue et maintient un premier diamètre intérieur intermédiaire (D₁) dudit joint d'étanchéité élastomère (44) qui est supérieur audit diamètre intérieur fermé dudit joint d'étanchéité élastomère (44) mais qui est inférieur audit diamètre intérieur ouvert (D₀) dudit joint d'étanchéité élastomère (44) ;
dans lequel ladite pluralité de gradins (106, 108, 110) inclut un deuxième gradin (108) pour engager séquentiellement ladite une extrémité respective desdites secondes extrémités de bras de levier (69) de ladite paire de bras de levier (63) et pour établir une seconde position intermédiaire pendant une rotation séquentielle de ladite bague de verrouillage (90) depuis ladite première position intermédiaire, dans lequel ladite seconde position intermédiaire effectue et maintient un second diamètre intérieur intermédiaire (D₂) dudit joint d'étanchéité élastomère (44) qui est supérieur audit premier diamètre intérieur intermédiaire (D₁) mais qui est inférieur audit diamètre intérieur ouvert (D₀) dudit joint d'étanchéité élastomère (44) ; et
dans lequel ladite pluralité de gradins (106, 108, 110) inclut un troisième gradin (110) pour engager séquentiellement ladite une extrémité respective desdites secondes extrémités de bras de levier (69) de ladite paire de bras de levier (63) pour effectuer une troisième augmentation incrémentale de la distance D entre ladite plaque à piston (28) et ladite bride de logement (38) pour établir ladite position ouverte et maintenir ledit diamètre intérieur ouvert (D₀) dudit joint d'étanchéité élastomère (44).

8. Assemblage de valvule médicale (10) selon la revendication 3, comprenant en outre :
ledit logement de valvule (32) qui définit des pas de vis de valvule (112) disposés de manière adjacente à ladite seconde extrémité de logement de valvule (36) ; et
ladite base (92) de ladite bague de verrouillage (90) qui définit des pas de vis de verrouillage (114) s'étendant de manière concentrique autour dudit orifice de base (96) et engagée par vissage avec lesdits pas de vis de valvule (112) pour établir ladite connexion en rotation entre ladite bague de verrouillage (90) et ledit logement de valvule (32).

9. Assemblage de valvule médicale (10) selon la revendication 8, dans lequel ledit élément de verrouillage (96) inclut un recouvrement (116) s'étendant de manière concentrique autour dudit axe (A) et s'étendant depuis ladite base (92) jusqu'à une extrémité de recouvrement (118) pour définir une surface intérieure de recouvrement (120) pour établir ladite surface de verrouillage (98), et dans lequel ladite bague de verrouillage (90) peut être mise en rotation autour desdits pas de vis de valvule (114) pour faire avancer axialement ladite bague de verrouillage (90) depuis ladite position fermée dans laquelle ladite extrémité de recouvrement (118) est disposée dans une relation d'espacement avec lesdites secondes extrémités de bras de levier (69) de ladite paire de bras de levier (63) jusqu'à ladite position ouverte dans laquelle ladite surface intérieure de recouvrement (120) comprime radialement ladite paire de bras de levier (63).

10. Assemblage de valvule médicale (10) selon la revendication 9, dans lequel ledit recouvrement (116) a un premier diamètre de recouvrement (D_{S1}) adjacent à ladite base (92) et un second diamètre de recouvrement (D_{S2}) adjacent à ladite extrémité de recouvrement (118), ledit second diamètre de recouvrement (D_{S2}) étant supérieur audit premier diamètre de recouvrement (D_{S1}) pour définir une surface de verrouillage en rampe (98) pour comprimer radialement de manière incrémentale et séquentielle ladite paire de bras de levier (63) pendant un déplacement de ladite bague de verrouillage (90) depuis ladite position fermée jusqu'à ladite position ouverte.

11. Assemblage de valvule médicale (10) selon la revendication 10, dans lequel ladite surface de verrouillage en rampe (98) établit une pluralité de positions intermédiaires pendant un déplacement axial de ladite bague de verrouillage (90) depuis ladite position fermée jusqu'à ladite position ouverte, et dans lequel chacune de la pluralité de positions intermédiaires effectue une augmentation incrémentale et séquentielle de ladite distance (D) entre ladite plaque à piston (28) et ladite bride de logement (32) et maintient un diamètre intérieur intermédiaire respectif dudit joint d'étanchéité élastomère (44) qui est supérieur à une position précédente de ladite pluralité de positions intermédiaires et qui est inférieur à une position suivante de ladite pluralité de positions intermédiaires.

12. Assemblage de valvule médicale (10) selon la revendication 11, dans lequel ladite seconde extrémité de logement de valvule (36) définit un arrêt (122) et lesdits pas de vis de valvule (112) s'étendent depuis ledit arrêt (122) et se terminent au niveau d'une lèvre (124), et dans lequel ladite base (92) de ladite bague de verrouillage (90) est disposée dans une relation de butée avec ladite lèvre (124) dans ladite position fermée et est avancée axialement dans une relation de butée avec ledit arrêt (122) pour établir ladite position ouverte.
